# FASCICULE DE BREVET EUROPEEN

(11) **EP 3 801 012 B1**
(45) Date de publication et mention de la délivrance du brevet: **24.04.2024**
(21) Numéro de dépôt: 19742433.6
(22) Date de dépôt: 31.05.2019
(51) Int. Cl.: A01K 67/033

(54) **MILIEU DE PONTE POUR INSECTES COMPORTANT UN SUBSTRAT TEXTURANT**
EIERLEGEMEDIUM FÜR INSEKTEN MIT EINEM TEXTURIERENDEN SUBSTRAT
EGG-LAYING MEDIUM FOR INSECTS, COMPRISING A TEXTURING SUBSTRATE

(30) Priorité: 01.06.2018 FR 1854799
(43) Date de publication de la demande: 14.04.2021
(73) Titulaire: Ynsect, 91058 Evry-Courcouronnes Cedex (FR)
(72) Inventeur: MATHIEU, Marianne, 77140 SAINT PIERRE LES NEMOURS (FR); ESCALANTE NOGUERA, Pedro, 91260 JUVISY SUR ORGE (FR); BERRO, Fabrice, 75001 PARIS (FR); DU JONCHAY, Thibault, 60710 CHEVRIÈRES (FR); BEREZINA, Nathalie, 1772 Järfälla (SE)
(74) Mandataire: Santarelli
(86) Numéro de dépôt international: PCT/FR2019/051284
(87) Numéro de publication internationale: WO 2019/229396

(56) Documents cités:
- EP-A2- 2 265 132
- CN-A- 106 359 306
- KR-A- 20130 046 658
- Fao: "texture du sol", , 31 juillet 2013 (2013-07-31), XP055551113, Extrait de l'Internet: URL:http://blog.ac-versailles.fr/formation capa/public/le_sol/LE_SOL__2_.pdf [extrait le 2019-02-04]
- HOUSE H L: "AN ARTIFICIAL HOST: ENCAPSULATED SYNTHETIC MEDIUM FOR IN VITRO OVIPOSITION AND REARING THE ENDOPARASITOID ITOPLECTIS CONQUISITOR (HYMENOPTERA: ICHNEUMONIDAE)", CANADIAN ENTOMOLOGIST, OTTAWA, CA, vol. 110, no. 3, 1 mars 1978 (1978-03-01), pages 331-333, XP001027170, ISSN: 0008-347X

## Description

La présente invention concerne l'élevage d'insectes et plus particulièrement l'élevage des coléoptères et/ou des lépidoptères. Elle concerne plus particulièrement un milieu de ponte et un bac de ponte et leurs utilisations, notamment dans un procédé de collecte d'oeufs d'insectes.

L'élevage d'insectes connaît un essor important ces dernières années. La production d'insectes présente de nombreux intérêts, que ce soit pour l'agro-industrie, les insectes constituant une source intéressante de protéines, ou dans d'autres domaines industriels, les insectes étant également une source de chitine, transformable en chitosan, dont les applications sont nombreuses : cosmétique, médicale et pharmaceutique, diététique et alimentaire, traitement de l'eau, etc.

L'élevage d'insectes à l'échelle industrielle suppose de pouvoir faire se reproduire de manière efficace les insectes.

Le plus souvent, en élevage, les femelles insectes pondent des oeufs dans leur milieu nutritif. Ces oeufs, souvent très petits, éclosent quelques semaines après la ponte. Or, il arrive que les larves qui viennent d'éclore dévorent les oeufs non encore éclos. D'où la nécessité de bien séparer les oeufs de la population larvaire afin de maintenir une production élevée.

Mais il existe donc un besoin pour un procédé de collecte d'oeufs efficace à l'échelle industrielle. Le document CN-A-106359306 vise à améliorer la quantité d'oeufs pondus par des criquets ainsi que la collecte des ces oeufs.

KR20130046658 est relatif à une méthode pour recueillir les oeufs d'un insecte *Tenebrio molitor* (ou *T. molitor*) comprenant un récipient et un filet de filtration amovible, la méthode comprenant notamment les étapes suivantes : introduire dans un récipient de la farine de céréales, introduire les individus au stade adulte dans le filet de filtration amovible, inciter les femelles à pondre des oeufs de manière à ce que ceux-ci soient collés à la paroi du récipient, et récupérer les oeufs d'une part et, grâce au filet de filtration amovible, les femelles d'autre part.

Cependant, ce document ne décrit pas clairement comment les femelles sont incitées à pondre les oeufs de manière à ce que ceux-ci soient collés à la paroi du récipient. De plus, une telle méthode n'est pas adaptée à une échelle industrielle, impliquant productivité, notamment surfacique, élevée.

La présente invention vise à proposer un procédé de collecte d'oeufs d'insectes permettant de pallier les inconvénients ci-avant. Par « oeufs d'insectes », on vise plus particulièrement des oeufs d'insectes isolés, c'est-à-dire n'étant pas sous forme d'amas appelés oothèques.

Le travail des inventeurs leur a permis d'élaborer ce procédé de collecte, qui nécessite l'utilisation d'un milieu de ponte spécifique.

L'invention concerne donc un milieu de ponte pour insectes comportant :
- au moins 75 % en poids d'un substrat texturant non consommable sous forme de particules, au moins 85% en poids desdites particules ayant une taille inférieure à 0,5 mm, ledit substrat texturant non consommable ayant une humidité comprise entre 0 et 10%,
- au moins 5% en poids d'un substrat nutritif ayant une humidité comprise entre 3 et 60%,
les pourcentages en poids étant donnés sur le poids total de milieu de ponte pour insectes.

On notera que, dans le cadre de la présente demande, et sauf stipulation contraire, les gammes de valeurs indiquées s'entendent bornes incluses.

Par « substrat nutritif », on vise un substrat nutritif ou un mélange de substrats nutritifs, destiné à être consommé par les insectes.

Le substrat nutritif peut présenter une humidité comprise entre 30 et 60%. Dans ce cas, un apport d'eau complémentaire n'est pas nécessaire.

Avantageusement, le substrat nutritif a une humidité comprise entre 3 et 40%. Dans ce cas, un apport d'eau complémentaire peut être nécessaire. Cet apport d'eau complémentaire peut par exemple être effectué via l'ajout d'eau sous forme pulvérisée et/ou via l'introduction d'un gel aqueux et optionnellement nutritif. Lorsqu'un gel aqueux et optionnellement nutritif est utilisé pour effectuer l'apport d'eau, celui-ci est introduit à une quantité d'au moins 0,5% en poids, de préférence au moins 1% en poids sur le poids total du milieu de ponte.

De préférence, l'invention concerne un milieu de ponte pour insectes comportant :
- au moins 75% en poids d'un substrat texturant non consommable sous forme de particules, au moins 85% en poids desdites particules ayant une taille inférieure à 0,5 mm, ledit substrat texturant non consommable ayant une humidité comprise entre 0 et 10%,
- au moins 5% en poids d'un substrat nutritif ayant une humidité comprise entre 3 et 40%, et
- au moins 2% en poids d'un gel aqueux et optionnellement nutritif,
les pourcentages en poids étant donnés sur le poids total de milieu de ponte pour insectes.

Avantageusement, le substrat nutritif a une humidité comprise entre 3 et 15%.

Par « substrat texturant non consommable », on vise un substrat texturant qui n'est pas consommé par les insectes, c'est-dire que les insectes ne vont pas l'ingérer afin de se nourrir. En particulier, ce substrat texturant non consommable peut être réutilisé.

La taille de particules est une caractéristique bien connue de l'homme du métier, qui permet de caractériser des compositions telles que, par exemple, des poudres, des moutures.

La granulométrie est l'étude de la distribution de la taille des particules d'une composition. Les techniques d'analyse granulométrique sont bien connues de l'homme du métier. A titre d'exemple, on pourra se référer à la publication suivant : « La granulométrie de l'aliment : principe, mesure et obtention ; INRA Prod. Anim., 2000, 13 (2), 81-97 ».

Par « particules ayant une taille inférieure à Y », on entend des particules qui passent à travers un tamis ayant un vide de maille de Y.

Préférentiellement, au moins 90% en poids des particules du substrat texturant non consommable a une taille inférieure à 0,5 mm.

Les inventeurs ont mis en évidence que les adultes consomment beaucoup moins d'aliment que ce qui doit leur être apporté pour qu'ils assurent une reproduction efficace. Or, un défaut d'aliment a pour conséquence que les adultes se reproduisent moins. Ainsi, les inventeurs ont mis au point un milieu de ponte comportant deux substrats, chacun exerçant une fonction distincte : une fonction de texturation qui est assuré par le substrat texturant non consommable et une fonction nutritive qui est assuré par le substrat nutritif. La combinaison de ces deux substrats permet en particulier d'obtenir de bonnes performances de reproduction. De plus, le substrat texturant n'étant pas consommé par les insectes adultes, il peut être réutilisé.

De par le choix des différents paramètres exposés ci-avant, le milieu de ponte pour insectes selon l'invention permet notamment :
- La séparation aisée et propre des oeufs d'insectes des autres constituants du milieu de ponte : adultes, restes d'adultes morts, excréments (frass), substrats et éventuellement du gel. Ceci permet notamment d'augmenter les densités dans la production, en obtenant une meilleure productivité par unité de surface ;
- De résoudre les problèmes de développement des parasites opportunistes (mites, mouches, etc.) qui peuvent coloniser les substrats nutritifs consommés de manière insuffisante par les adultes et petites larves. La présence de substrats non consommés pendant une période étendue est principalement due aux difficultés de concentrer les insectes du stade de l'œuf jusqu'à la phase des larves de 20 mg. Cela est également effectué par un contrôle de l'humidité ;
- D'augmenter les performances de reproduction. Cette augmentation peut s'expliquer notamment par le choix du milieu de ponte, qui favorise notamment une diminution de la consommation accidentelle des oeufs par les adultes. Or, usuellement, dans les conditions densifiées, la probabilité d'occurrence de tels évènements est plus élevée. Le choix du milieu de ponte permet une collecte facilitée des oeufs, collecte dont la fréquence peut être augmentée et qui limite ainsi ce phénomène ;
- Tout en maintenant une productivité élevée.

De préférence, ce milieu de ponte comporte :
- de 81 % à 89% en poids d'un substrat texturant non consommable sous forme de particules, au moins 85% en poids desdites particules ayant une taille inférieure à 0,5 mm, ledit substrat texturant non consommable ayant une humidité comprise entre 0 et 10%,
- de 3% à 5% en poids d'un gel aqueux et optionnellement nutritif, et
- de 8% à 14% en poids d'un substrat nutritif ayant une humidité comprise entre 3 et 15%,
les pourcentages en poids étant donnés sur le poids total de milieu de ponte pour insectes.

Avantageusement, dans le milieu de ponte selon l'invention, le substrat texturant non consommable est un substrat minéral, plastique et/ou organique.

De préférence, le substrat minéral est choisi parmi la perlite, la zéolite et la vermiculite.

De préférence, le substrat plastique est choisi parmi le polypropylène, le polystyrène et le polyéthylène.

Dans le cas où le substrat texturant non consommable est un substrat minéral ou plastique, ce substrat texturant non consommable est sous forme de particules, au moins 85% en poids desdites particules ayant une taille inférieure à 0,5 mm. Une telle taille de particules permet une séparation aisée du substrat des excrétions générées par les insectes adultes lors de l'utilisation du milieu de ponte.

De préférence, le substrat organique non consommable par les insectes est choisi parmi les excrétions (« frass ») de larves d'insectes.

Dans ce cas, au moins 85% en poids des particules du substrat texturant non consommable a une taille de particule comprise entre 0,3 et 0,5 mm (borne excluse).

En effet, il est avantageux que les particules du substrat texturant non consommable aient une taille de particule supérieure à 0,3 et inférieure à 0,5 mm. Notamment, il est préférable qu'au moins 50% des particules du substrat texturant non consommable aient une taille de particule supérieure à 0,3 et inférieure à 0,5 mm.

Par « particules ayant une taille supérieure à X », on entend des particules qui sont retenues par (ne passent pas à travers) un tamis ayant un vide de maille de X.

De préférence, les excrétions de larves d'insectes sont issues de larves de *Tenebrio molitor, Tribolium castaneum, Tribolium confusum, Dermestes ater, Dermestes magister, Alphitobius diaperinus, Zophobas morios, Oryzaephilus surinamensis, Cryptolestes ferrugineus, Trogoderma granarium, Gnathocerus cornutus, Tenebroides mauritanicus et Ephestia kuehniella.*

Plus préférentiellement, dans le milieu de ponte l'invention, le substrat texturant est constitué d'excrétions hygiénisées de larves d'insectes.

Par "hygiénisé", on entend un procédé comportant une étape de nettoyage physique des excrétions de larves d'insectes (notamment, par isolation et récupération des particules d'excrétion de larves d'insectes) et une étape de réduction de la charge microbienne, par exemple par un traitement sanitaire tel qu'un traitement thermique.

Avantageusement, dans le milieu de ponte selon l'invention, le gel aqueux et optionnellement nutritif comporte :
- au moins 90% en poids d'une solution aqueuse,
- de 0,3 à 2% en poids d'un agent gélifiant, et
- de 0,1 à 5% en poids d'un agent conservateur,
les pourcentages en poids étant exprimés sur le poids total du gel.

De préférence, le gel aqueux et optionnellement nutritif a une teneur en eau supérieure à 50%, préférentiellement supérieure à 70%, plus préférentiellement encore supérieure à 90% en poids sur le poids total de gel.

Selon un premier mode de réalisation du gel aqueux et optionnellement nutritif, la solution aqueuse est constituée d'eau.

Selon un second mode de réalisation du gel aqueux et optionnellement nutritif, le gel est également nutritif et la solution aqueuse peut comporter, outre de l'eau, un coproduit liquide de l'agro-industrie. De préférence, l'agro-industrie est choisie parmi les industries de l'amidonnerie, de la féculerie, de la malterie, de production de bioéthanol, du sucre, de la fermentation, de la brasserie, de la distillation et l'industrie laitière. De préférence, le coproduit liquide de l'agro-industrie est choisi parmi la liste constituée par les solubles de céréales, les solubles de maïs, les solubles de blé, les solubles de pois, les solubles de manioc, les solubles de betterave à sucre, les solubles de canne à sucre, les solubles de distillerie de céréales, les solubles de distillerie de blé, les solubles de distillerie de maïs, les solubles de distillerie de pois, les solubles de distillerie du manioc, les vinasses, les mélasses, les crèmes de levures, les lactosérums et leurs dérivés concentrés notamment le perméat, ou leurs mélanges. Plus préférentiellement, le coproduit liquide de l'agro-industrie est choisi parmi un soluble de distillerie ou un mélange d'un soluble de distillerie avec un autre coproduit liquide.

Avantageusement, l'agent gélifiant choisi parmi le groupe constitué par la gomme de xanthane, la gomme de caroube, la gomme de guar, ou leur mélange. De préférence, l'agent gélifiant est un mélange de gomme de xanthane et de gomme de caroube ou de gomme de xanthane et de gomme de guar.

Le gel aqueux et optionnellement nutritif peut également comporter des levures, des vitamines et/ou des probiotiques.

Avantageusement, le gel aqueux et optionnellement nutritif a une force de gel d'au moins 20 g/cm², préférentiellement, 30 g/cm², plus préférentiellement 50 g/cm².

Cette force de gel permet l'obtention d'un gel solide et de structure peu visqueuse, qui ne sera pas asséché par la présence de particules fines susceptible d'adhérer au gel.

De préférence, dans le milieu de ponte selon l'invention, le substrat nutritif est sous forme de particules dont au moins 75% en poids a une taille supérieure à 0,5 mm. Plus préférentiellement, le substrat nutritif est sous forme de particules dont la taille est supérieure à 0,5 mm.

De préférence, au moins 75% en poids des particules du substrat nutritif a une taille supérieure à 0,7 mm.

Plus préférentiellement, au moins 75% en poids des particules du substrat nutritif a une taille supérieure à 0,7 et inférieure à 1,5 mm.

Plus préférentiellement encore, au moins 85% en poids des particules du substrat nutritif présente les tailles de particules mentionnées ci-avant.

De préférence, le substrat nutritif n'est donc pas une farine, dont la taille de particules est notablement inférieure.

Avantageusement, le substrat nutritif est un coproduit ou un mélange de coproduits solide de l'industrie des céréales, des oléagineux, des oléoprotéagineux ou des protéagineux.

Un coproduit est une matière inévitable créée au cours d'un processus de fabrication d'un produit d'intérêt.

Par industrie des céréales, on vise plus notamment les industries de l'amidonnerie, de la féculerie, de la malterie, de production de bioéthanol, de la fermentation, de la brasserie et de la distillation, telle que l'industrie du blé, de l'orge ou du maïs.

Par industrie des oléagineux, des oléoprotéagineux ou des protéagineux, on vise plus particulièrement l'industrie du colza, du tournesol, du lin, du soja ou du pois.

De préférence, le coproduit solide est un coproduit de l'industrie du blé et notamment du remoulage de blé ou du son de blé. Plus préférentiellement, le coproduit solide est du son de blé. A titre d'exemple, 75% en poids des particules de son de blé a une taille de particule supérieure à 0,8 et inférieure à 1,4 mm.

Alternativement, des drêches sèches de distillerie avec soluble (« Dried Distilled Grains with Solubles ») peuvent être utilisées.

Le milieu de ponte selon l'invention est avantageusement disposé sur le fond d'un contenant afin de former un bac de ponte.

Un bac de ponte pas compris dans l'invention, comportant un contenant et, sur un fond dudit contenant :
- de 0,12 à 6,75 g/cm² d'un substrat texturant non consommable sous forme de particules, au moins 85% en poids desdites particules ayant une taille inférieure à 0,5 mm, ledit substrat texturant non consommable ayant une humidité comprise entre 0 et 10%,
- de 0,0014 à 0,105 g/cm²/j d'un substrat nutritif ayant une humidité comprise entre 3 et 60%, et,
- optionnellement de 0,0016 à 0,095 g/cm²/j d'un gel aqueux et optionnellement nutritif.

Les quantités surfaciques sont exprimées par rapport à la surface du fond du contenant. Un tel bac de ponte est adapté aux insectes.

On notera que les quantités en g/cm²/j dépendent du temps de séjour en jour (j) des insectes.

De préférence, la surface du fond du contenant du bac de ponte comporte :
- de 0,148 à 6,2 g/cm² d'un substrat texturant non consommable sous forme de particules, au moins 85% en poids desdites particules ayant une taille inférieure à 0,5 mm, ledit substrat texturant non consommable ayant une humidité comprise entre 0 et 10%,
- de 0,0028 à 0,105 g/cm²/j d'un substrat nutritif ayant une humidité comprise entre 3 et 40%, et
- de 0,0022 à 0,08 g/cm²/j d'un gel aqueux et optionnellement nutritif.

Alternativement, le bac de ponte selon l'invention comporte un contenant et, sur un fond dudit contenant :
- de 0,12 à 6,75 g/cm² d'un substrat texturant non consommable sous forme de particules, au moins 85% en poids desdites particules ayant une taille inférieure à 0,5 mm, ledit substrat texturant non consommable ayant une humidité comprise entre 0 et 10%,
- de 0,01 à 0,75 g/cm² d'un substrat nutritif ayant une humidité comprise entre 3 et 60%, et,
- optionnellement de 0,006 à 0,325 g/cm² d'un gel aqueux et optionnellement nutritif.

Un tel bac de ponte est adapté aux insectes et plus particulièrement pour un séjour de 7j des insectes, séjour au cours duquel la teneur en gel sera renouvelée une fois.

De préférence, la surface du fond du contenant du bac de ponte comporte :
- de 0,148 à 6,2 g/cm² d'un substrat texturant non consommable sous forme de particules, au moins 85% en poids desdites particules ayant une taille inférieure à 0,5 mm, ledit substrat texturant non consommable ayant une humidité comprise entre 0 et 10%,
- de 0,02 à 0,75 g/cm² d'un substrat nutritif ayant une humidité comprise entre 3 et 40%, et
- de 0,0074 à 0,275 g/cm² d'un gel aqueux et optionnellement nutritif.

De préférence, notamment, dans le bac de ponte :
- au moins 90% en poids des particules du substrat texturant non consommable a une taille inférieure à 0,5 mm ;
- le substrat texturant non consommable est un substrat minéral, plastique et/ou organique, ledit substrat texturant non consommable pouvant présenter les caractéristiques particulières, avantageuses et préférées telles qu'indiquées pour le milieu de ponte ci-avant ; en particulier, le substrat texturant non consommable est un substrat organique non consommable choisi parmi les excrétions hygiénisées (« frass ») de larves d'insectes ;
- le gel aqueux et optionnellement nutritif comporte :
   ∘ au moins 90% en poids d'une solution aqueuse,
   ∘ de 0,3 à 2% en poids d'un agent gélifiant, et
   ∘ de 0,1 à 5% en poids d'un agent conservateur,
   les pourcentages en poids étant exprimés sur le poids total du gel ; ledit gel aqueux et optionnellement nutritif pouvant présenter les caractéristiques particulières, avantageuses et préférées telles qu'indiquées pour le milieu de ponte ci-avant ; en particulier, le gel aqueux et optionnellement nutritif a une teneur en eau supérieure à 50%, préférentiellement supérieure à 70%, plus préférentiellement encore supérieure à 90% en poids sur le poids total de gel ;
- le substrat nutritif a une humidité comprise entre 3 et 15% ; et/ou
- le substrat nutritif est sous forme de particules dont au moins 75% en poids a une taille supérieure à 0,5 mm, préférentiellement supérieure à 0,7 mm et plus préférentiellement encore supérieure à 0,7 et inférieure à 1,5 mm ; ledit substrat nutritif pouvant présenter les caractéristiques particulières, avantageuses et préférées telles qu'indiqués pour le milieu de ponte ci-avant ; en particulier, le substrat nutritif est du remoulage de blé, du son de blé et/ou des drêches de distillerie avec solubles.

Avantageusement, le substrat texturant non consommable, le substrat nutritif et le gel aqueux et optionnellement nutritif du bac de ponte selon l'invention proviennent d'un milieu de ponte selon l'invention. En particulier, les teneurs en substrat texturant non consommable, en substrat nutritif et en gel aqueux et optionnellement nutritif du bac de ponte selon l'invention sont celles indiquées ci-avant.

L'invention se rapporte également à l'utilisation d'un milieu de ponte selon l'invention, d'un bac de ponte selon l'invention pour l'élevage de coléoptères et/ou lépidoptères.

Par coléoptères et/ou lépidoptères, on vise plus particulièrement les coléoptères et lépidoptères appartenant aux familles des Tenebrionidae, Melolonthidae, Dermestidae, Coccinellidae, Cerambycidae, Carabidae, Buprestidae, Cetoniidae, Dryophthoridae, Silvanidae, Trogoderma, Laemophloeidae, Trogossitidae, Pyralidae ou leurs mélanges.

Plus préférentiellement, il s'agit des coléoptères et/ou lépidoptères suivants : *Tenebrio molitor*, *Tenebrio obscurus*, *Tribolium castaneum, Tribolium confusum, Dermestes ater, Dermestes magister, Alphitobius diaperinus, Zophobas morio, Rhynchophorus ferrugineus, Oryzaephilus surinamensis, Cryptolestes ferrugineus, Trogoderma granarium, Gnathocerus cornutus, Tenebroides mauritanicus et Ephestia kuehniella.*

Plus préférentiellement, le milieu de ponte selon l'invention et le bac de ponte selon l'invention sont mis en oeuvre dans l'élevage de coléoptères, en particulier des familles des Tenebrionidae, Melolonthidae, Dermestidae, Coccinellidae, Cerambycidae, Carabidae, Buprestidae, Cetoniidae et Dryophthoridae.

Plus préférentiellement, il s'agit des coléoptères *Tenebrio molitor*, *Tenebrio obscurus*, *Tribolium castaneum, Alphitobius diaperinus, Zophobas morio, Rhynchophorus ferrugineus,* ou leur mélange, et plus particulièrement dans l'élevage de *Tenebrio molitor.*

L'invention concerne enfin un procédé d'obtention d'oeufs d'insectes comportant les étapes de :
- obtention d'un bac de ponte par la fourniture d'un contenant et le remplissage dudit contenant avec :
   ∘ un substrat texturant non consommable sous forme de particules, au moins 85% en poids desdites particules ayant une taille inférieure à 0,5 mm, ledit substrat texturant non consommable ayant une humidité comprise entre 0 et 10%,
   ∘ un substrat nutritif ayant une humidité comprise entre 3 et 60%, et,
   ∘ optionnellement un gel aqueux et optionnellement nutritif, pour obtenir un bac de ponte,
- introduction d'insectes adultes dans le bac de ponte, et
une étape subséquente de collecte des oeufs d'insectes.

Le substrat nutritif peut présenter une humidité comprise entre 30 et 60%. Avantageusement, le substrat nutritif a une humidité comprise entre 3 et 40%, préférentiellement, entre 3 et 15%.

Dans le procédé d'obtention d'oeufs d'insectes selon l'invention, l'étape de remplissage du contenant par un substrat texturant non consommable est effectuée par apport de 0,12 à 6,75 g/cm² de substrat texturant non consommable dans le contenant.

Préférentiellement, l'étape de remplissage du contenant par un substrat texturant non consommable est effectuée par un apport de 0,148 à 6,2 g/cm² de substrat texturant non consommable.

Avantageusement, l'apport de substrat texturant non consommable dans le contenant s'effectue sur une hauteur de 1 à 5 cm, préférentiellement sur une hauteur de 2 à 4 cm.

De préférence, dans le procédé d'obtention d'oeufs d'insectes selon l'invention, l'étape de remplissage du contenant par un substrat nutritif est effectuée par apport de 0,0014 à 0,105 g/cm²/j de substrat nutritif.

La quantité de substrat nutritif en g/cm²/j dépend du temps de séjour en jour (j) des insectes dans le bac de ponte. Ce temps de séjour correspond au nombre de jours écoulé depuis l'introduction des insectes dans le bac de ponte et l'étape de collecte des oeufs.

Pour un temps de séjour de 7j, la quantité de substrat nutritif est de 0,01 à 0,75 g/cm², préférentiellement, de 0,02 à 0,105 g/cm².

Préférentiellement, dans le procédé d'obtention d'oeufs d'insectes selon l'invention, l'étape de remplissage du contenant comporte l'introduction d'un gel aqueux et optionnellement nutritif à un apport de 0,0016 à 0,095 g/cm²/j.

La quantité de gel aqueux et optionnellement nutritif en g/cm²/j dépend également du temps de séjour en jour (j) des insectes dans le bac de ponte.

Pour un temps de séjour de 3,5 jours, la quantité de gel aqueux et optionnellement nutritif est de 0,006 à 0,325 g/cm², préférentiellement, de 0,0074 à 0,275 g/cm².

Avantageusement, dans le procédé d'obtention d'oeufs d'insectes selon l'invention, l'étape d'introduction des insectes adultes dans le bac de ponte est effectuée à une densité surfacique comprise entre 0,01 et 1,0 g/cm², préférentiellement à une densité surfacique comprise entre 0,02 à 0,75 g/cm².

Préférentiellement, le procédé d'obtention d'oeufs d'insectes selon l'invention comporte les étapes suivantes :
- obtention d'un bac de ponte par la fourniture d'un contenant et le remplissage d'un fond dudit contenant avec :
   ∘ 0,148 à 6,2 g/cm² d'un substrat texturant non consommable sous forme de particules, au moins 85% en poids desdites particules ayant une taille inférieure à 0,5 mm, ledit substrat texturant non consommable ayant une humidité comprise entre 0 et 10%,
   ∘ 0,0028 à 0,105 g/cm²/j d'un substrat nutritif ayant une humidité comprise entre 3 et 40%,
   ∘ 0,0022 à 0,08 g/cm²/j d'un gel aqueux et optionnellement nutritif,
- introduction d'insectes adultes dans le bac de ponte, et
une étape subséquente de collecte des oeufs d'insectes.

Alternativement, le procédé d'obtention d'oeufs d'insectes selon l'invention comporte les étapes suivantes :
- obtention d'un bac de ponte par la fourniture d'un contenant et le remplissage d'un fond dudit contenant avec :
   ∘ 0,148 à 6,2 g/cm² d'un substrat texturant non consommable sous forme de particules, au moins 85% en poids desdites particules ayant une taille inférieure à 0,5 mm, ledit substrat texturant non consommable ayant une humidité comprise entre 0 et 10%,
   ∘ 0,02 à 0,75 g/cm² d'un substrat nutritif ayant une humidité comprise entre 3 et 40%,
   ∘ 0,0074 à 0,275 g/cm² d'un gel aqueux et optionnellement nutritif,
- introduction d'insectes adultes dans le bac de ponte, et
une étape subséquente de collecte des oeufs d'insectes.

Avantageusement, dans les procédés d'obtention d'oeufs selon l'invention, le substrat nutritif a une humidité comprise entre 3 et 15%.

Un tel procédé est adapté pour un séjour de 7j des insectes dans le bac de ponte, séjour au cours duquel la teneur en gel sera renouvelée une fois, par exemple au cours du jour 3, le jour 1 étant le jour d'introduction des insectes adultes dans le bac de ponte.

Selon un mode de réalisation particulièrement avantageux, l'étape subséquente de collecte des oeufs du procédé d'obtention d'oeufs d'insectes selon l'invention est effectuée au moyen d'une étape de tri automatisé.

L'étape de tri automatisé permet une séparation aisée des différents éléments contenus dans le bac de ponte.

Cette étape de tri automatisé peut être effectuée aux moyens de dispositifs tels que des tamiseurs à nutation oscillatoire ou des tamiseurs vibratoires linéaires.

Ces dispositifs permettent de séparer aisément différentes fractions, classées ci-après par taille croissante :
- La fraction de substrat texturant non consommable,
- La fraction d'excrétions d'insectes adultes,
- La fraction d'oeufs d'insectes,
- La fraction des restes d'insectes adultes morts,
- La fraction des insectes adultes.

A l'issue de l'étape de tri automatisé, la récupération de la fraction d'oeufs d'insectes permet la collecte des oeufs.

La fraction d'oeufs pouvant comporter de particules de substrat nutritif, il est possible d'effectuer une étape supplémentaire de séparation afin d'obtenir des oeufs purs et propres. Dans ce cas, la fraction d'oeufs subit une séparation densimétrique avec une vitesse de ventilation adaptée à la quantité d'oeufs pour permettre l'obtention d'oeufs purs et propres.

Alternativement, il est possible d'utiliser la fraction d'oeufs telle quelle.

Par ailleurs, le tri automatisé permet également de séparer et collecter les insectes adultes. Les adultes vivants peuvent ensuite être séparés des adultes morts à l'aide d'une colonne densimétrique. Une fois séparés, les insectes adultes vivants peuvent être réutilisés pour peupler un nouveau bac de ponte selon l'invention.

Enfin, la récupération de la fraction de substrat texturant non consommable permet sa réutilisation, après hygiénisation.

Préférentiellement, l'étape de collecte du procédé d'obtention d'oeufs d'insectes selon l'invention est effectuée tous les 2 à 3 jours.

Une récolte tous les 2 à 3 jours permet d'augmenter les performances de ponte d'au moins 20%.

De préférence, notamment, dans le procédé d'obtention d'oeufs d'insectes selon l'invention :
- au moins 90% en poids des particules du substrat texturant non consommable a une taille inférieure à 0,5 mm ;
- le substrat texturant non consommable est un substrat minéral, plastique et/ou organique, ledit substrat texturant non consommable pouvant présenter les caractéristiques avantageuses et préférées telles qu'indiquées pour le milieu et le bac de ponte ci-avant ; en particulier, le substrat texturant non consommable est un substrat organique non consommable choisi parmi les excrétions hygiénisées (« frass ») de larves d'insectes ;
- le gel aqueux et optionnellement nutritif comporte :
   ∘ au moins 90% en poids d'une solution aqueuse,
   ∘ de 0,3 à 2% en poids d'un agent gélifiant, et
   ∘ de 0,1 à 5% en poids d'un agent conservateur,
   les pourcentages en poids étant exprimés sur le poids total du gel ; ledit gel aqueux et optionnellement nutritif pouvant présenter les caractéristiques avantageuses et préférées telles qu'indiquées pour le milieu et le bac de ponte ci-avant ; en particulier, le gel aqueux et optionnellement nutritif a une teneur en eau supérieure à 50%, préférentiellement supérieure à 70%, plus préférentiellement encore supérieure à 90% en poids sur le poids total de gel ;
- le substrat nutritif a une humidité comprise entre 3 et 15% ; et/ou
- le substrat nutritif est sous forme de particules dont au moins 75% en poids a une taille supérieure à 0,5 mm, préférentiellement supérieure à 0,7 mm et plus préférentiellement encore supérieure à 0,7 et inférieure à 1,5 mm ; ledit substrat nutritif pouvant présenter les caractéristiques avantageuses et préférées telles qu'indiqués pour le milieu et le bac de ponte ci-avant ; en particulier, le substrat nutritif est du remoulage de blé, du son de blé et/ou des drêches de distillerie avec solubles.

Le procédé d'obtention d'oeufs d'insectes selon l'invention est particulièrement adapté pour l'élevage de coléoptères et/ou lépidoptères. Les coléoptères et/ou lépidoptères préférés sont tels qu'indiqués ci-avant, et plus préférentiellement, le procédé d'obtention d'oeufs d'insectes selon l'invention est particulièrement adapté pour l'élevage de *T*. *molitor.*

Avantageusement, le substrat texturant non consommable, le substrat nutritif et le gel aqueux et optionnellement nutritif utilisés dans le procédé selon l'invention proviennent d'un milieu de ponte selon l'invention. En particulier, les teneurs en substrat texturant non consommable, en substrat nutritif et en gel aqueux et optionnellement nutritif du bac de ponte selon l'invention sont celles indiquées ci-avant.

D'autres caractéristiques et avantages de l'invention apparaîtront dans les exemples qui suivent, donnés à titre illustratif, avec référence aux figures, qui représentent respectivement :
- La Figure 1 est un schéma d'un bac de ponte selon l'invention,
- La Figure 2 est un schéma représentant la séparation des différentes fractions lors de l'étape automatisée de tri et de collecte des oeufs d'insectes,
- La Figure 3 est composées de deux photographies représentant deux fractions d'oeufs issues d'un tri d'un bac de ponte : sur la figure de gauche, la taille des particules de substrat texturant n'a pas été sélectionnée tandis que sur la figure de droite, la fraction d'oeufs a été triée selon la méthode selon l'invention, le substrat texturant ayant une granulométrie adaptée au tri des oeufs (excrétions hygiénisées de larves de *Tenebrio molitor,* particules inférieures à 0,5mm), et
- La Figure 4 est un schéma de mise en oeuvre « en continu » du procédé d'obtention d'oeufs d'insectes.

### Exemple I : Préparation d'un milieu de ponte et d'un bac de ponte

Le milieu de ponte est préparé à l'aide des trois composants suivants :
- Substrat texturant non consommable : des excrétions (« frass ») hygiénisées de larves de *T. molitor.* Il s'agit plus particulièrement d'excrétions de jeunes larves de *T. molitor* (larves de 1-50 mg de masse individuelle), dont la granulométrie est la suivante :
   ▪ 91,84% en poids des particules d'excrétion ont une taille comprise entre 0,30 et 0,50 mm,
   ▪ 5,83% en poids des particules d'excrétion ont une taille comprise entre 0,25 et 0,30 mm, et
   ▪ 2,33% en poids des particules d'excrétion ont une taille comprise entre 0,50 et 0,80 mm
- Substrat nutritif : du son de blé, ayant une granulométrie comprise entre 0,8 et 1,4 mm et une humidité d'environ 11%.
- Gel : un gel aqueux est préparé à partir de 98,7% en poids d'eau, 1% en poids d'agent gélifiant (Flanogen) et 0,3% en poids de sorbate de potassium.

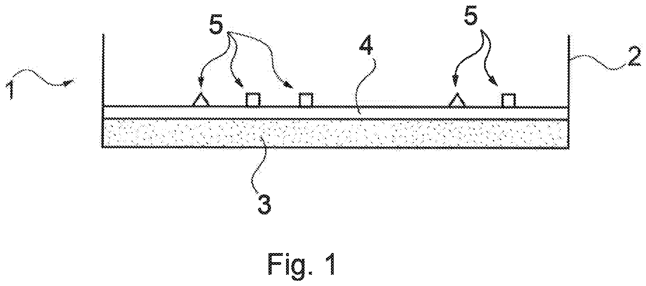

La Figure 1 représente un bac de ponte 1.

Dans un contenant 2 en plastique, on place :
- Un substrat texturant non consommable 3, à savoir 1500-2500 g d'excrétions (0,74-1,24 g/cm²) ;
- Un substrat nutritif 4, à savoir 200-300 g de son de blé (0,1-0,15 g/cm²) ;
- Un gel aqueux et optionnellement nutritif 5, à savoir 75-112,5 g de gel aqueux (0,037-0,055 g/cm²).

De préférence, les composants sont introduits dans l'ordre indiqués ci-avant.

On obtient ainsi un bac de ponte.

### Exemple II : Procédé d'obtention d'œufs d'insectes

### 1. Matériel

- De jeunes adultes *Tenebrio molitor* (âgés d'1 semaine)
- Des bacs de ponte de l'Exemple I
- Une salle d'élevage à température et humidité contrôlées
- Un tamiseur à nutation oscillatoire (« tumbler screening machine », Allgaier) ou un tamiseur vibratoire linéaire (« linear screening machine », Mogensen)

### 2. Méthodes

Tri des adultes : Pour peupler les bacs de ponte, une étape de tri à partir de ténébrions au stade nymphal peut être nécessaire. Les nymphes devenant adultes avec le temps, les adultes sont alors séparés des nymphes. Le tri entre les nymphes et les adultes est réalisé sur une période de temps qui ne dépasse pas 7 jours. Cette étape de tri permet ainsi d'obtenir une population adulte homogène (± 7 jours de différence d'âge au sein de la population) au sein du bac de ponte.

Création et peuplement du bac de ponte : Le bac de ponte est créé comme indiqué à l'Exemple I. Les adultes peuvent être soit issus de l'étape de tri ci-avant, soit issus d'un ancien bac de ponte. En effet, la population d'adultes pour la ponte est gardée pendant plusieurs semaines, par exemple 8 semaines tandis que les séjours des adultes dans un bac de ponte peuvent durer de 2 à 14 jours. Dès lors, à l'issue d'un séjour, les adultes sont à nouveau triés, notamment pour retirer les adultes morts et garder les adultes vivants, puis ces derniers sont placés à nouveau à la densité optimale d'adultes dans un bac de ponte.

### • Ténébrions adultes : 0,02-0,75 g/cm²

Une fois les bacs de ponte peuplés d'adultes, ils sont conservés dans une salle d'élevage ayant entre 50% et 90% d'humidité relative. Il peut être utile de réalimenter les bacs de ponte en gel aqueux. Typiquement, on apporte une quantité de gel aqueux (0,0074-0,275 g/cm²) 2 fois par semaine (soit un apport initial et un apport subséquent, effectué 3,5 jours après l'apport initial). Les quantités de matières sont calculées selon la surface du bac d'élevage.

Tri/Collecte des oeufs : La fréquence de collecte des oeufs peut être adaptée entre 2 à 7 jours. Dans le présent Exemple, la collecte des oeufs a été effectuée au bout d'une période de 7 jours. Le jour 7, le bac de ponte est récupéré dans la salle d'élevage et son contenu est déversé dans un tamiseur. Le tamiseur possède un ensemble de tamis qui permet la séparation en fonction de leur taille, des différentes fractions du contenu du bac de ponte.

L'étape de tri et de collecte des oeufs a été testée avec deux types de machines différentes, un tamiseur vibratoire linéaire et un tamiseur à nutation oscillatoire. Les deux machines ont donné de bons résultats lors de l'étape de collecte.

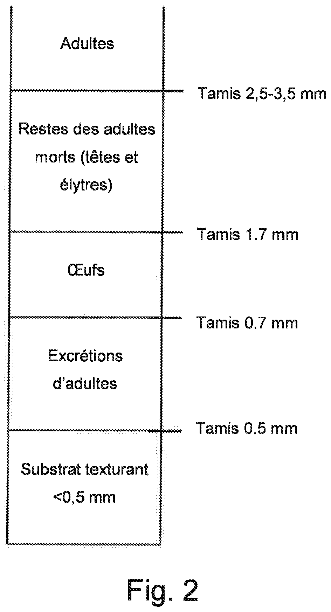

Sur la Figure 2, est décrite la séparation des différentes fractions en fonctions de leur taille :
- Fraction des insectes adultes : cette fraction correspond aux particules ne passant pas à travers (ou retenues par) un tamis ayant un vide de maille d'au plus 2,5 mm. Cette fraction comporte les adultes vivants et morts. Au cours d'une semaine de ponte d'oeufs dans des excrétions (substrat texturant) et du son de blé (substrat nutritif), une mortalité d'environ 10% des individus est constatée. Les adultes vivants sont ensuite séparés des adultes morts à l'aide d'une séparation densimétrique. Les adultes vivants sont à nouveau déposés dans des bacs de ponte.
- Fraction des restes d'adultes morts : cette fraction correspond aux particules passant à travers un tamis ayant un vide de maille d'au plus 2,5 mm et retenues par un tamis ayant un vide de maille de 1,7 mm. Cette fraction comporte des parties d'adultes morts (têtes, pattes, etc.).
- Fraction d'oeufs d'insectes : cette fraction correspond aux particules passant à travers un tamis ayant un vide de maille de 1,7 mm et retenues par un tamis ayant un vide de maille de 0,7 mm. Cette fraction comporte les oeufs. La quantité d'oeufs obtenus dépendra des conditions du bac de ponte (densité de la population, substrats, gel). Dans les conditions de cet Exemple, une moyenne de 25,9 ± 5,68 œufs/cm² ou 0,0186 ± 0,0046 g d'œufs/cm² est obtenue. Les oeufs obtenus après l'étape de tamisage sont mélangés avec des particules de substrat nutritif, quelques excrétions d'adultes et des restes d'adultes morts. En effet, généralement, après le tri, la fraction d'oeufs d'insectes comporte encore entre 50 et 60% en poids de particules, excrétions et restes (gros déchets).

- Fraction excrétions d'insectes adultes : cette fraction correspond aux particules passant à travers un tamis ayant un vide de maille de 0,7 mm et retenues par un tamis ayant un vide de maille de 0,5 mm. Cette fraction comporte les excrétions d'adultes.
- Fraction substrat texturant non consommable : cette fraction correspond aux particules passant à travers un tamis ayant un vide de maille de 0,5 mm. Le substrat texturant peut alors être potentiellement réutilisé après un traitement sanitaire.
- Enfin, lorsque l'apport de gel aqueux est effectué dans les quantités indiquées ci-avant, celui-ci est généralement entièrement consommé par les insectes. Si le gel aqueux n'est pas consommé, un tamis de maille de 5 mm peut être utilisé, afin de récupérer les morceaux de gel séché.

La récupération de la fraction d'oeufs d'insectes permet la collecte des oeufs. Comme indiqué ci-avant, cette fraction comprend encore entre 50 et 60% en poids de particules, excrétions et restes (gros déchets). Dès lors, elle peut être utilisée telle quelle, ou après une étape supplémentaire de séparation afin d'obtenir une fraction d'oeufs purs et propres. Dans ce cas la fraction d'oeufs d'insectes subit une séparation densimétrique, telle qu'une séparation sur colonne densimétrique, avec une vitesse de ventilation adaptée à la quantité d'oeufs pour permettre l'obtention d'oeufs purs et propres. La fraction d'oeufs purs et propres comporte alors de 65 à 75% en poids d'oeufs, une part importante des 25 à 35% en poids restant étant de fines particules de substrat nutritif.

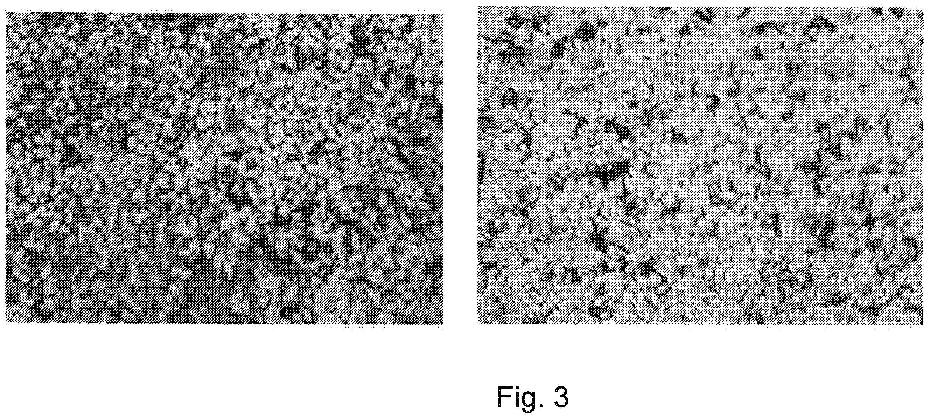

Sur la Figure 3, sont représentées deux fractions d'oeufs issues d'un tri d'un bac de ponte. Sur la figure de gauche, la taille des particules de substrat texturant n'a pas été sélectionnée tandis que sur la figure de droite, la fraction d'oeufs a été triée selon la méthode selon l'invention décrite ci-avant, le substrat texturant ayant une granulométrie adaptée au tri des oeufs particules inférieures à 0,5mm, ce qui conduit à une fraction d'oeufs de pureté supérieure.

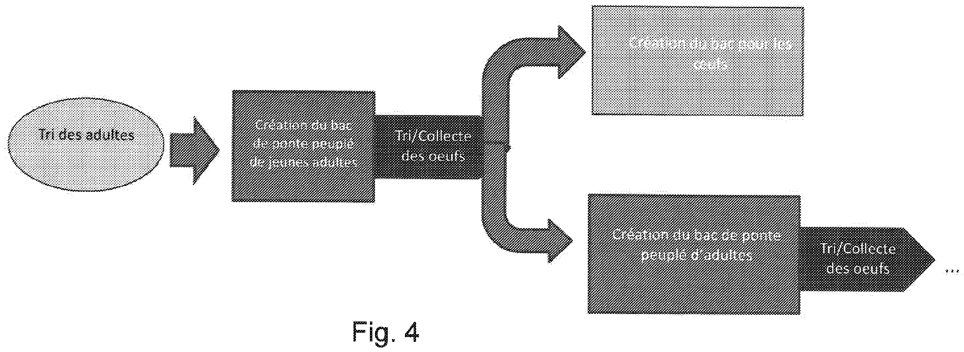

Sur la Figure 4, est représenté un schéma de mise en oeuvre « en continu » du procédé d'obtention d'oeufs d'insectes. Cette Figure est plus amplement détaillée ci-après :
Tri des adultes : voir la description ci-avant de cette étape.
Bac de pontes peuplé de jeunes adultes : comme indiqué ci-avant un bac de ponte est créé puis peuplé de jeunes adultes issus du tri des adultes.
Tri/Collecte des oeufs : voir la description ci-avant de cette étape.
Création du bac pour les oeufs : Ce bac peut ensuite être peuplé avec la fraction oeufs issu de la collecte ci-avant, en prenant en compte le fait que la masse d'oeufs purs est de 55% pour adapter la densité souhaitée d'oeufs, ou avec des oeufs purs et propres issus de l'étape de séparation supplémentaire. Les oeufs vont éclore 6 à 10 jours après création du bac pour les oeufs afin de donner des larves.
Bac de pontes peuplé d'adultes : comme indiqué ci-avant un bac de ponte est créé puis peuplé d'adultes récupérés à l'issus de l'étape de tri et collecte des oeufs.

## Revendications

1. Milieu de ponte pour insectes comportant :
- au moins 75 % en poids d'un substrat texturant non consommable sous forme de particules, au moins 85% en poids desdites particules ayant une taille inférieure à 0,5 mm, ledit substrat texturant non consommable ayant une humidité comprise entre 0 et 10%,
- au moins 5% en poids d'un substrat nutritif ayant une humidité comprise entre 3 et 60%,
les pourcentages en poids étant donnés sur le poids total de milieu de ponte pour insectes.

2. Milieu de ponte pour insectes selon la revendication 1, dans lequel le substrat texturant non consommable est constitué d'excrétions hygiénisées de larves d'insectes.

3. Milieu de ponte pour insectes selon la revendication 1 ou 2, dans lequel le substrat nutritif est sous forme de particules dont la taille est supérieure à 0,5 mm.

4. Milieu de ponte pour insectes l'une quelconque des revendications 1 à 3, dans lequel le substrat nutritif est un coproduit ou un mélange de coproduits solide de l'industrie des céréales, des oléagineux, des oléoprotéagineux ou des protéagineux.

5. Milieu de ponte pour insectes selon l'une quelconque des revendications 1 à 4, dans lequel le substrat nutritif a une humidité comprise entre 3 et 40%.

6. Milieu de ponte pour insectes selon la revendication 5, comportant en outre au moins 2% en poids d'un gel aqueux et optionnellement nutritif sur le poids total de milieu de ponte pour insectes.

7. Milieu de ponte pour insectes selon la revendication 6, dans lequel le substrat nutritif a une humidité comprise entre 3 et 15%.

8. Milieu de ponte pour insectes selon la revendication 6 ou 7, dans lequel le gel aqueux et optionnellement nutritif comporte :
- au moins 90% en poids d'une solution aqueuse,
- de 0,3 à 2% en poids d'un agent gélifiant, et
- de 0,1 à 5% en poids d'un agent conservateur,
les pourcentages en poids étant exprimés sur le poids total du gel.

9. Bac de ponte comportant un contenant et, sur le fond dudit contenant, un milieu de ponte selon l'une quelconque des revendications 1 à 8.

10. Bac de ponte selon la revendication 9 comportant sur le fond du contenant un milieu de ponte selon l'une quelconque des revendications 1 à 4 comprenant :
- de 0,12 à 6,75 g/cm² de substrat texturant non consommable sous forme de particules, au moins 85% en poids desdites particules ayant une taille inférieure à 0,5 mm, ledit substrat texturant non consommable ayant une humidité comprise entre 0 et 10%,
- de 0,01 à 0,75 g/cm² de substrat nutritif ayant une humidité comprise entre 3 et 60%, et,
- optionnellement de 0,006 à 0,325 g/cm² d'un gel aqueux et optionnellement nutritif.

11. Utilisation d'un milieu de ponte selon l'une quelconque des revendications 1 à 8, ou d'un bac de ponte selon l'une des revendications 9 ou 10, pour l'élevage de coléoptères et/ou lépidoptères.

12. Procédé d'obtention d'oeufs d'insectes comportant les étapes de :
- obtention d'un bac de ponte par la fourniture d'un contenant et le remplissage dudit contenant avec :
∘ un substrat texturant non consommable sous forme de particules, au moins 85% en poids desdites particules ayant une taille inférieure à 0,5 mm, ledit substrat texturant non consommable ayant une humidité comprise entre 0 et 10%,
∘ un substrat nutritif ayant une humidité comprise entre 3 et 60%, et
∘ optionnellement, un gel aqueux et optionnellement nutritif, pour obtenir un bac de ponte,
- introduction d'insectes adultes dans le bac de ponte, et
une étape subséquente de collecte des oeufs d'insectes.

13. Procédé d'obtention d'oeufs d'insectes selon la revendication 12, dans lequel l'étape de remplissage du contenant par un substrat texturant non consommable est effectuée par apport de 0,12 à 6,75 g/cm² de substrat texturant non consommable dans le contenant.

14. Procédé d'obtention d'oeufs d'insectes selon la revendication 12 ou 13, dans lequel l'étape de remplissage du contenant par un substrat nutritif est effectuée par apport de 0,0014 à 0,105 g/cm²/j de substrat nutritif.

15. Procédé d'obtention d'oeufs d'insectes selon l'une quelconque des revendications 12 à 14, dans lequel l'étape de remplissage du contenant comporte l'introduction d'un gel aqueux et optionnellement nutritif, à un apport de 0,0016 à 0,095 g/cm²/j.

16. Procédé d'obtention d'oeufs d'insectes selon l'une quelconque des revendications 12 à 15, dans lequel l'étape d'introduction des insectes adultes dans le bac de ponte est effectuée à une densité surfacique comprise entre 0,01 et 1,0 g/cm².

17. Procédé d'obtention d'oeufs d'insectes selon l'une quelconque des revendications 12 à 16, dans laquelle l'étape subséquente de collecte des oeufs est effectuée au moyen d'une étape de tri automatisé.

18. Procédé d'obtention d'oeufs d'insectes selon l'une quelconque des revendications 12 à 17, dans lequel les insectes adultes sont des coléoptères et/ou des lépidoptères.

## Patentansprüche

1. Eiablagemedium für Insekten, umfassend:
- mindestens 75 Gew.-% eines nicht verbrauchbaren Texturierungssubstrats in Form von Partikeln, wobei mindestens 85 Gew.-% der Partikel eine Größe von weniger als 0,5 mm aufweisen, wobei das nicht verbrauchbare Texturierungssubstrat einen Feuchtigkeitsgehalt zwischen 0 und 10 % aufweist,
- mindestens 5 Gew.-% eines Nährsubstrats, das einen Feuchtigkeitsgehalt zwischen 3 und 60 % aufweist,
wobei die Gewichtsprozente bezogen auf das Gesamtgewicht des Eiablagemediums für Insekten angegeben sind.

2. Eiablagemedium für Insekten nach Anspruch 1, wobei das nicht verbrauchbare Texturierungssubstrat aus den hygienisierten Ausscheidungen von Insektenlarven besteht.

3. Eiablagemedium für Insekten nach Anspruch 1 oder 2, wobei das Nährsubstrat in Form von Partikeln vorliegt, deren Größe größer als 0,5 mm ist.

4. Eiablagemedium für Insekten nach einem der Ansprüche 1 bis 3, wobei das Nährsubstrat ein Nebenprodukt oder eine feste Mischung von Nebenprodukten aus der Getreide-, Ölsaaten-, Eiweißölpflanzen- oder Eiweißpflanzenindustrie ist.

5. Eiablagemedium für Insekten nach einem der Ansprüche 1 bis 4, wobei das Nährsubstrat einen Feuchtigkeitsgehalt zwischen 3 und 40 % aufweist.

6. Eiablagemedium für Insekten nach Anspruch 5, das ferner mindestens 2 Gew.-% eines wässrigen und gegebenenfalls nahrhaften Gels bezogen auf das Gesamtgewicht des Eiablagemediums für Insekten umfasst.

7. Eiablagemedium für Insekten nach Anspruch 6, wobei das Nährsubstrat einen Feuchtigkeitsgehalt zwischen 3 und 15 % aufweist.

8. Eiablagemedium für Insekten nach Anspruch 6 oder 7, wobei das wässrige und gegebenenfalls nahrhafte Gel Folgendes umfasst:
- mindestens 90 Gew.-% einer wässrigen Lösung,
- 0,3 bis 2 Gew.-% eines Geliermittels, und
- 0,1 bis 5 Gew.-% eines Konservierungsmittels,
wobei die Gewichtsprozente bezogen auf das Gesamtgewicht des Gels ausgedrückt werden.

9. Eiablageschale, die einen Behälter und auf dem Boden des Behälters ein Eiablagemedium nach einem der Ansprüche 1 bis 8 umfasst.

10. Eiablageschale nach Anspruch 9, die auf dem Boden des Behälters ein Eiablagemedium nach einem der Ansprüche 1 bis 4 umfasst, das Folgendes beinhaltet:
- 0,12 bis 6,75 g/cm² eines nicht verbrauchbaren Texturierungssubstrats in Form von Partikeln, wobei mindestens 85 Gew.-% der Partikel eine Größe von weniger als 0,5 mm aufweisen, wobei das nicht verbrauchbare Texturierungssubstrat einen Feuchtigkeitsgehalt zwischen 0 und 10 % aufweist,
- 0,01 bis 0,75 g/cm² eines Nährsubstrats, das einen Feuchtigkeitsgehalt zwischen 3 und 60 % aufweist, und
- gegebenenfalls 0,006 bis 0,325 g/cm² eines wässrigen und gegebenenfalls nahrhaften Gels.

11. Verwendung eines Eiablagemediums nach einem der Ansprüche 1 bis 8 oder einer Eiablageschale nach einem der Ansprüche 9 oder 10 zur Aufzucht von Käfern und/oder Schmetterlingen.

12. Verfahren zum Gewinnen von Insekteneiern, das die folgenden Schritte umfasst:
- Erhalten einer Eiablageschale durch das Bereitstellen eines Behälters und das Befüllen des Behälters mit Folgendem:
o einem nicht verbrauchbaren Texturierungssubstrat in Form von Partikeln, wobei mindestens 85 Gew.-% der Partikel eine Größe von weniger als 0,5 mm aufweisen, wobei das nicht verbrauchbare Texturierungssubstrat einen Feuchtigkeitsgehalt zwischen 0 und 10 % aufweist,
o einem Nährsubstrat, das einen Feuchtigkeitsgehalt zwischen 3 und 60 % aufweist, und
o gegebenenfalls einem wässrigen und gegebenenfalls nahrhaften Gel,
um eine Eiablageschale zu erhalten,
- Einbringen erwachsener Insekten in die Eiablageschale und einen anschließenden Schritt des Sammelns der Insekteneier.

13. Verfahren zum Gewinnen von Insekteneiern nach Anspruch 12, wobei der Schritt des Befüllens des Behälters mit einem nicht verbrauchbaren Texturierungssubstrat durch eine Menge von 0,12 bis 6,75 g/cm² eines nicht verbrauchbaren Texturierungssubstrats in den Behälter durchgeführt wird.

14. Verfahren zum Gewinnen von Insekteneiern nach Anspruch 12 oder 13, wobei der Schritt des Befüllens des Behälters mit einem Nährsubstrat durch eine Menge von 0,0014 bis 0,105 g/cm²/Tag eines Nährsubstrats durchgeführt wird.

15. Verfahren zum Gewinnen von Insekteneiern nach einem der Ansprüche 12 bis 14, wobei der Schritt des Befüllens des Behälters das Einbringen eines wässrigen und gegebenenfalls nahrhaften Gels in einer Menge von 0,0016 bis 0,095 g/cm²/Tag umfasst.

16. Verfahren zum Gewinnen von Insekteneiern nach einem der Ansprüche 12 bis 15, wobei der Schritt des Einbringens der erwachsenen Insekten in die Eiablageschale mit einer Flächendichte zwischen 0,01 und 1,0 g/cm² durchgeführt wird.

17. Verfahren zum Gewinnen von Insekteneiern nach einem der Ansprüche 12 bis 16, wobei der anschließende Schritt des Sammelns der Insekteneier mittels eines Schritts des automatisierten Sortierens durchgeführt wird.

18. Verfahren zum Gewinnen von Insekteneiern nach einem der Ansprüche 12 bis 17, wobei die erwachsenen Insekten Käfer und/oder Schmetterlinge sind.

## Claims

1. Egg-laying medium for insects comprising:
- at least 75% by weight of a non-consumable texturing substrate in the form of particles, at least 85% by weight of said particles having a size smaller than 0.5 mm, said non-consumable texturing substrate having a moisture content comprised between 0 and 10%,
- at least 5% by weight of a nutrient substrate having a moisture content comprised between 3 and 60%,
wherein the percentages by weight are given in relation to the total weight of egg-laying medium for insects.

2. Egg-laying medium for insects according to claim 1, in which the non-consumable texturing substrate is constituted by sanitized excrement of insect larvae.

3. Egg-laying medium for insects according to claim 1 or 2, in which the nutrient substrate is in the form of particles, the size of which is larger than 0.5 mm.

4. Egg-laying medium for insects according to any one of claims 1 to 3, in which the nutrient substrate is a solid co-product or mixture of co-products from the cereal, oilseed, protein-oil crop or protein crop industry.

5. Egg-laying medium for insects according to any one of claims 1 to 4, in which the nutrient substrate has a moisture content comprised between 3 and 40%.

6. Egg-laying medium for insects according to claim 5, also comprising at least 2% by weight of an aqueous and optionally nutritional gel in relation to the total weight of egg-laying medium for insects.

7. Egg-laying medium for insects according to claim 6, in which the nutrient substrate has a moisture content comprised between 3 and 15%.

8. Egg-laying medium for insects according to claim 6 or 7, in which the aqueous and optionally nutritional gel comprises:
- at least 90% by weight of an aqueous solution,
- 0.3 to 2% by weight of a gelling agent, and
- 0.1 to 5% by weight of a preservative,
wherein the percentages by weight are expressed in relation to the total weight of the gel.

9. Laying tray comprising a container and, on the bottom of said container, an egg-laying medium according to any one of claims 1 to 8.

10. Laying tray according to claim 9 comprising on the bottom of the container an egg-laying medium according to any one of claims 1 to 4 comprising:
- 0.12 to 6.75 g/cm² of a non-consumable texturing substrate in the form of particles, at least 85% by weight of said particles having a size smaller than 0.5 mm, said non-consumable texturing substrate having a moisture content comprised between 0 and 10%,
- 0.01 to 0.75 g/cm² of a nutrient substrate having a moisture content comprised between 3 and 60%, and
- optionally 0.006 to 0.325 g/cm² of an aqueous and optionally nutritional gel.

11. Use of an egg-laying medium according to any one of claims 1 to 8 or a laying tray according to claim 9 or 10, for breeding coleopterans and/or lepidopterans.

12. Method for obtaining insect eggs, comprising the steps of:
- obtaining a laying tray by providing a container and filling said container with:
∘ a non-consumable texturing substrate in the form of particles, at least 85% by weight of said particles having a size smaller than 0.5 mm, said non-consumable texturing substrate having a moisture content comprised between 0 and 10%,
∘ a nutrient substrate having a moisture content comprised between 3 and 60%, and
∘ optionally an aqueous and optionally nutritional gel,
in order to obtain a laying tray,
- introducing adult insects into the laying tray, and
a subsequent step of collecting the insect eggs.

13. Method for obtaining insect eggs according to claim 12, in which the step of filling the container with a non-consumable texturing substrate is effected by supplying 0.12 to 6.75 g/cm² of non-consumable texturing substrate into the container.

14. Method for obtaining insect eggs according to claim 12 or 13, in which the step of filling the container with a nutrient substrate is effected by supplying 0.0014 to 0.105 g/cm²/d of nutrient substrate.

15. Method for obtaining insect eggs according to any one of claims 12 to 14, in which the step of filling the container comprises introducing an aqueous and optionally nutritional gel in a supply of from 0.0016 to 0.095 g/cm²/d.

16. Method for obtaining insect eggs according to any one of claims 12 to 15, in which the step of introducing the adult insects into the laying tray is effected in an areal density comprised between 0.01 and 1.0 g/cm².

17. Method for obtaining insect eggs according to any one of claims 12 to 16, in which the subsequent step of collecting the eggs is effected by means of an automated sorting step.

18. Method for obtaining insect eggs according to any one of claims 12 to 17, in which the adult insects are coleopterans and/or lepidopterans.
